(19) Europäisches Patentamt
European Patent Office
Office européen des brevets

(11) Veröffentlichungsnummer: **0 021 041**
**B1**

# (12) EUROPÄISCHE PATENTSCHRIFT

(45) Veröffentlichungstag der Patentschrift: **02.06.82**

(51) Int. Cl.³: **C 07 F 9/58**, A 01 N 57/16 // C07D213/64

(21) Anmeldenummer: **80102812.7**

(22) Anmeldetag: **21.05.80**

## (54) 6-Fluorpyridyl-(di)(thio)phosphorsäureester, Verfahren zu ihrer Herstellung und ihre Verwendung zur Bekämpfung von Schädlingen.

(30) Priorität: **15.06.79 DE 2924150**

(43) Veröffentlichungstag der Anmeldung: **07.01.81 Patentblatt 81/1**

(45) Bekanntmachung des Hinweises auf die Patenterteilung: **02.06.82 Patentblatt 82/22**

(84) Benannte Vertragsstaaten: **AT BE CH DE FR GB IT LI LU NL SE**

(56) Entgegenhaltungen:
**DE-A-2 358 760**
**US-A-3 743 648**
**US-A-3 810 902**

(73) Patentinhaber: **BASF Aktiengesellschaft, Carl-Bosch-Strasse 38, D-6700 Ludwigshafen (DE)**

(72) Erfinder: **Theobald, Hans, Dr., Parkstrasse 2, D-6703 Limburgerhof (DE)**
Erfinder: **Adolphi, Heinrich, Dr., Kalmitweg 11, D-6703 Limburgerhof (DE)**
Erfinder: **Eicken, Karl, Dr., Waldstrasse 63, D-6706 Wachenheim (DE)**
Erfinder: **Oeser, Heinz-Guenter, Dr., Friedrich-Burschell-Weg 19, D-6700 Ludwigshafen (DE)**

EP 0 021 041 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# 6-Fluorpyridyl-(di)(thio)phosphorsäureester, Verfahren zu ihrer Herstellung und ihre Verwendung zur Bekämpfung von Schädlingen

Die vorliegende Erfindung betrifft neue 6-Fluorpyridyl-phosphorsäurederivate, ein Verfahren zu ihrer Herstellung und Schädlingsbekämpfungsmittel, die diese Phosphorsäurederivate als Wirkstoffe enthalten, sowie ein Verfahren zur Bekämpfung von Schädlingen mit diesen Wirkstoffen.

Es ist bekannt, daß O,O-Dialkyl-O-halogenpyridyl-phosphorsäureester zur Bekämpfung von Schädlingen aus der Klasse der Insekten geeignet sind (US-PS 3 928 370). Weiterhin ist bekannt, daß O,O-Diäthyl-O-(6-fluorpyridyl-2)-thiophosphat nematizid wirksam ist (US-PS 3 810 902).

Es wurde gefunden, daß 6-Fluorpyridyl-(di)(thio)phosphorsäureester der Formel I

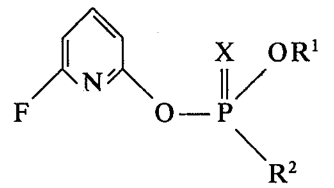

(I)

in der

X Sauerstoff oder Schwefel,
$R^1$ eine unverzweigte oder verzweigte Alkylgruppe mit bis zu 3 Kohlenstoffatomen und
$R^2$ eine unverzweigte oder verzweigte Alkylthiogruppe mit bis zu 6 Kohlenstoffatomen bedeuten,

Schädlinge aus der Klasse der Insekten und Nematoden wirksamer bekämpfen als bekannte Halogenpyridyl-phosphorsäurederivate.

Unverzweigte oder verzweigte Alkylreste für $R^1$ in Formel I sind die Methyl-, die Äthyl-, die n-Propyl- und i-Propylgruppe. Unverzweigte oder verzweigte Alkylthiogruppen für $R^2$ sind Methylthio, Äthylthio, n-Propylthio, i-Propylthio, n-Butylthio, i-Butylthio und sec.-Butylthio, tert.-Butylthio, 3-Methyl-n-butylthio, 2-Methyl-n-butylthio, 4-Methyl-n-pentylthio, 4-Äthyl-n-butylthio, Neopentylthio. Insbesondere bevorzugt sind Methyl und Äthyl für $R^1$, Propylthio- und Butylthioreste für $R^2$.

Man erhält die 6-Fluorpyridyl-(di)(thio)phosphorsäureester der Formel I dadurch, daß man Fluorpyridylderivate der Formel II mit (Di)(Thio)Phosphorsäureesterchloriden der Formel III entsprechend folgender Reaktionsgleichung umsetzt:

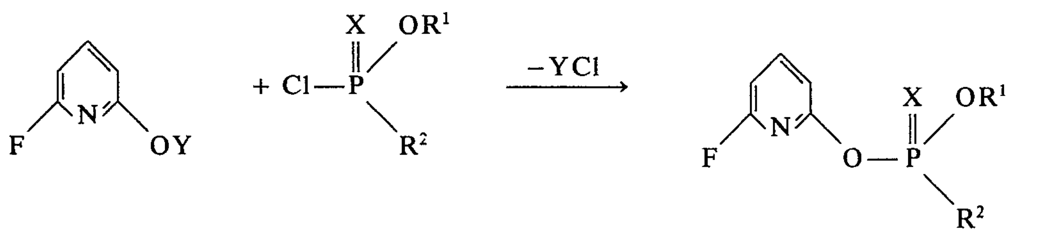


(II) (III) (I)

Dabei haben die Reste $R^1$, $R^2$ und X die oben angegebenen Bedeutungen und Y steht für Wasserstoff, ein Alkaliion oder ein Äquivalent Erdalkaliion.

Wird 2-Hydroxy-6-fluor-pyridin eingesetzt, kann die bei der Reaktion entstehende Chlorwasserstoffsäure beispielsweise durch Einblasen von Inertgas, wie z. B. Stickstoff, oder durch Zugabe von Basen aus dem Reaktionsgemisch entfernt werden. Geeignete Basen sind beispielsweise Alkali- und Erdalkalicarbonate und Hydroxide, Alkali- und Erdalkalihydrogencarbonate, Ammoniak und gegebenenfalls durch Alkylreste substituierte Amine.

Die Umsetzung wird im allgemeinen in gegenüber den Reaktionsteilnehmern inerten Lösungs- und Verdünnungsmitteln durchgeführt. Hierfür sind beispielsweise geeignet: Wasser; Äther, wie Tetrahydrofuran, Dioxan, Diglykoldimethyläther; Ketone, wie Aceton, Methyläthylketon, Diäthylketon; aromatische Kohlenwasserstoffe, wie Toluol, Xylole, Chlorbenzole; Nitrile, wie Acetonitril; Dimethylformamid; Dimethylsulfoxid. Auch Gemische dieser Lösungs- oder Verdünnungsmittel können verwendet werden. In einigen Fällen kann die Zugabe von Phasentransferkatalysatoren von Vorteil sein.

Zur Durchführung des Verfahrens setzt man die Ausgangsstoffe meist in äquimolaren Verhältnissen ein. Ein Überschuß der einen oder anderen Reaktionskomponente kann in einigen Fällen Vorteile bringen.

Die Reaktionstemperatur kann innerhalb eines größeren Bereiches variiert werden. Im allgemeinen arbeitet man bei Temperaturen im Bereich von 0 bis 150° C, vorzugsweise im Bereich von 20 bis 100° C.

Das als Ausgangsstoff verwendbare 2-Hydroxy-6-fluorpyridin der Formel II kann aus 2,6-Difluorpyridin, das aus Rec. trav. chim. Pays-Bas 81, 1058 (1962), bekannt ist, hergestellt werden. Die (Di)(Thio)Phosphorsäureesterchloride der Formel III können nach bekannten Verfahren hergestellt werden (Houben—Weyl, Methoden der organischen Chemie, Bd. 12/2, S. 621, 755, Georg Thieme-Verlag, Stuttgart, 1964).

Die folgenden Beispiele erläutern die Herstellung der neuen 6-Fluorpyridyl-(di)(thio)phosphorsäureester. Gewichtsteile verhalten sich zu Volumenteilen wie Kilogramm zu Liter.

Beispiel 1

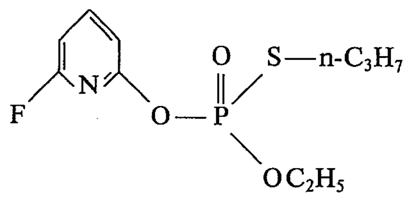

(Wirkstoff Nr. 1)

115 Gewichtsteile 2,6-Difluorpyridin in 500 Volumenteilen Toluol werden bei 80°C zu 120 Volumenteilen 50%iger wäßriger Natronlauge zugetropft. Dann wird das Reaktionsgemisch 5 Stunden lang bei 90 bis 100°C nachgerührt. Nach dem Abkühlen werden die Toluolphase abgetrennt und die Wasserphase mit konz. Chlorwasserstoffsäure angesäuert. Man äthert aus, trocknet die Ätherphase über Natrium, filtriert und engt ein. Der Rückstand besteht aus 2-Hydroxy-6-fluorpyridin vom Schmelzpunkt 127 bis 130°C.

11,2 Gewichtsteile 2-Hydroxy-6-fluorpyridin werden in 80 Volumenteilen Methanol mit 18 Volumenteilen 30%iger Natriummethylatlösung eine Stunde bei 50°C gerührt. Danach wird vom Lösungsmittel abrotiert und in 100 Volumenteilen Acetonitril aufgenommen. Nach Zugabe von 20,3 Gewichtsteilen O-Äthyl-S-n-propyl-phosphorsäurechlorid wird 7 Stunden bei 60°C gerührt. Nach dem Abkühlen wird filtriert und eingeengt. Der ölige Rückstand wird in Toluol aufgenommen und mit 5%iger Natriumhydrogencarbonatlösung und mit Wasser gewaschen. Nach dem Trocknen über Natriumsulfat wird eingeengt und bei 50°C/0,013 mbar andestilliert. Man erhält 26,5 Gewichtsteile eines hellgelben Öls.

$C_{10}H_{15}NPO_3SF$ (279):

| | | | | | | |
|---|---|---|---|---|---|---|
| Ber.: | C 43,0 | H 5,4 | N 5,0 | S 11,5 | P 11,1 | F 6,8 |
| Gef.: | C 43,3 | H 5,6 | N 5,5 | S 11,8 | P 10,7 | F 6,6 |

220-MHz-NMR-Spektrum ($CDCl_3$, $\delta$-Werte):

0,95 (3H); 1,4 (3H); 1,78 (2H); 3,02 (2H); 4,38 (2H); 6,82 (1H); 6,98 (1H); 7,9 (1H).

Analog können die in der folgenden Tabelle beschriebenen (Di)(Thio)Phosphorsäureester der Formel I hergestellt werden.

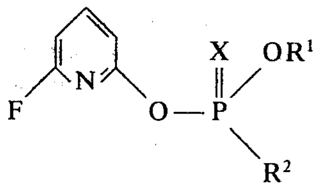

| Wirkstoff Nr. | X | $R^1$ | $R^2$ | NMR-Daten (in $CDCl_3$; $\delta$-Werte) |
|---|---|---|---|---|
| 2 | S | $C_2H_5$ | $n-C_3H_7S$ | (220 MHz), 0,95 (3H); 1,38 (3H); 1,7 (2H); 3,1 (2H); 4,35 (2H); 6,82 (1H); 7,03 (1H); 7,9 (1H) |
| 3 | O | $C_2H_5$ | $i-C_4H_9-S$ | (220 MHz), 0,98 (3H); 1,44 (3H); 1,98 (1H); 3,0 (2H); 4,5 (2H); 6,8 (1H); 7,1 (1H); 7,9 (1H); |
| 4 | O | $C_2H_5$ | $sec-C_4H_9-S$ | (220 MHz), 0,95 (3H); 1,3–1,5 (3H + 3H); 1,7 (2H); 3,5 (2H); 4,3 (2H); 6,8 (1H); 7,1 (1H); 7,85 (1H); |

Die erfindungsgemäßen 6-Fluorpyridyl-(di)(thio)phosphorsäureester der Formel I sind geeignet, Schädlinge aus der Klasse der Insekten und Nemathelminthes wirksam zu bekämpfen. Sie können im Pflanzenschutz sowie auf dem Hygiene-, Vorratsschutz- und Veterinärsektor eingesetzt werden.

Zu den schädlichen Insekten gehören aus der Ordnung der Schmetterlinge (Lepidoptera) beispielsweise

Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rückenzünsler), Ephestia kuehniella (Mehlmotte), Chilo suppressalis (Reisstengelbohrer), Galleria mellonella (Wachsmotte), Malacosoma neustria (Ringelspinner), Dendrolimus pini (Kiefernspinner), Thaumatopoea pityocampa (Pinienprozessionsspinner), Phalera bucephala (Mondfleck), Cheimatobia brumata (Kleiner Frostspanner), Hibernia defoliaria (Großer Frostspanner), Bupalus piniarus (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling);

aus der Ordnung der Käfer (Coleoptera) beispielsweise

Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varivestris (Mexikanischer Bohnenkäfer), Phyllopertha horticola (Junikäfer),

Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Otiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Wandgärtner);

aus der Ordnung der Zweiflügler (Diptera) beispielsweise

Mayetiola destructor (Hessenfliege), Dasyneura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeer-Fruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hyoscyami (Rübenfliege);

aus der Ordnung der Hautflügler (Hymenoptera) beispielsweise

Athalia rosae (Rübenblattwespe), Hoplocampa minuta (Pflaumensägewespe), Monomorium pharaonis (Pharaoameise), Solenopsis geminata (Feuerameise), Atta sexdens (Blattschneiderameise);

aus der Ordnung der Wanzen (Heteroptera) beispielsweise

Nezara viridula (Grüne Reiswanze), Eurygaster integriceps (Asiatische Getreidewanze), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);

aus der Ordnung der Pflanzensauger (Homoptera) beispielsweise

Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nastrutii (Kreuzdornblattlaus), Cerosipha gossypii (Gurkenblattlaus), Sappaphis mali (Rosige Apfellaus), Sappaphis mala (Mehlige Birnblattlaus), Dysaphis radicola (Mehlige Apfelfalterlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenlaus), Macrosiphon rosae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);

aus der Ordnung der Termiten (Isoptera) beispielsweise

Reticulitermes lucifugus;

aus der Ordnung der Geradflügler (Orthoptera) beispielsweise

Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Malanoplus femur-rubrum (Rotbeinige Heuschecke), Blatta orientalis (Küchenschabe),

Blattella germanica (Deutsche Schabe), Periplaneta americana (Amerikanische Schabe), Blabera gigantea (Riesenschabe).

Zur Klasse der Nemathelminthes zählen beispielsweise Wurzelgallennematoden, z. B. Meloidogyne incognita, Meloidogyne hapla, Meloidogyne javanica, Zysten bildende Nematoden, z. B. Heterodere rostochiensis, Heterodera schachtii, Heterodera avenae, Heterodera glycines, Heterodera trifolii, Stock- und Blattälchen, z. B. Ditylenchus dipsaci, Ditylenchus destructor, Pratylenchus neglectus, Pratylenchus penetrans, Pratylenchus goodeyi, Pratylenchus curvitatus sowie Tylenchorhynchus dibius, Tylenchorhynchus claytoni, Rotylenchus robustus, Heliocotylenchus multicinctus, Radopholus similis, Belonolaimus longicaudatus, Longidorus elongatus, Trichodorus primitivus.

Die Wirkstoffe können als solche, in Form ihrer Formulierungen oder den daraus bereiteten Anwendungsmöglichkeiten, z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Äthanol, Propanol, Butanol, Chloroform, Tetrachlorkohlenstoff, Cyclohexanol, Cyclohexanon, Chlorbenzol, Isophoron, stark polare Lösungsmittel, z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylenoctylphenoläther, äthoxyliertes Isooctylphenol, Octylphenol, Nonylphenon, Alkylphenolpolyglykoläther, Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Fomulierungen sind:

I. 3 Gewichtsteile O-Äthyl-S-n-propyl-O-(6-fluorpyridyl-2)-thiophosphat werden mit 97 Gewichtsteilen feinteiligem Kaolin innig vermischt. Man erhält auf diese Weise ein Stäubemittel, das 3 Gew.-% des Wirkstoffes enthält.

II. 30 Gewichtsteile O-Äthyl-S-i-propyl-O-(6-fluorpyridyl-2)-thiophosphat werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffes mit guter Haftfähigkeit.

III. 20 Gewichtsteile O-Äthyl-S-n-propyl-O-(6-fluorpyridyl-2)-dithiophosphat werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffes enthält.

IV. 20 Gewichtsteile O-Äthyl-S-i-butyl-O-(6-fluorpyridyl-2)-phosphat werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffes enthält.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%. Die Wirkstoffe werden in Form dieser Formulierungen oder in Form daraus herstellbarer anwendungsfertiger Zubereitungen angewendet. Die Wirkstoffkonzentrationen in solchen anwendungsfertigen Zubereitungen können in größeren Bereichen variiert werden. Im allgemeinen liegen sie zwischen 0,0001 und 10%, vorzugsweise zwischen 0,01 und 5%.

Die Wirkstoffe können auch mit gutem Erfolg im Ultra-Low-Volume-Verfahren (ULV) verwendet werden; dabei ist es möglich, Formulierungen mit über 95 Gew.-% Wirkstoff oder sogar den Wirkstoff ohne Zusätze auszubringen.

Zu den Wirkstoffen können Öle verschiedenen Typs, Herbizide, Fungizide, andere Insektizide, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1 : 10 bis 10 : 1 zugemischt werden.

Beispielsweise können folgende Mittel zugemischt werden:

1,2-Dibrom-3-chlorpropan,
1,3-Dichlorpropen,
1,3-Dichlorpropen + 1,2-Dichlorpropan,
1,2-Dibrom-äthan,
2-sec.-Butyl-phenyl-N-methylcarbamat,
o-Chlorphenyl-N-methylcarbamat,
3-Isopropyl-5-methylphenyl-N-methylcarbamat,
o-Isopropoxyphenyl-N-methylcarbamat,
3,5-Dimethyl-4-methylmercapto-phenyl-N-methylcarbamat,
4-Dimethylamino-3,5-xylyl-N-methylcarbamat,
2-(1,3-Dioxolan-2-yl)-phenyl-N-methylcarbamat,
1-Naphthyl-N-methylcarbamat,
2,3-Dihydro-2,2-dimethyl-benzofuran-7-yl-N-methylcarbamat,
2,2-Dimethyl-1,3-benzodioxol-4-yl-N-methylcarbamat,
2-Dimethylamino-5,6-dimethyl-4-pyrimidinyl-dimethylcarbamat,
2-Methyl-2-(methylthio)-propionaldehyd-O-(methylcarbamoyl)-oxim,
S-Methyl-N-[(methylcarbamoyl)-oxy]-thio-acetimidat,
Methyl-N',N'-dimethyl-N-[(methylcarbamoyl)-oxy]-1-thiooxamidat,
N-(2-Methyl-4-chlor-phenyl)-N',N'-dimethylformamidin,
Tetrachlorthiophen,
1-(2,6-Difluor-benzoyl)-3-(4-chlor-phenyl)-harnstoff,
O,O-Dimethyl-O-(p-nitrophenyl)-phosphorthioat,
O,O-Diäthyl-O-(p-nitrophenyl)-phosphorthioat,
O-Äthyl-O-(p-nitrophenyl)-phenyl-phosphonothioat,
O,O-Dimethyl-O-(3-methyl-4-nitrophenyl)-phosphorthioat,
O,O-Diäthyl-O-(2,4-dichlorphenyl)-phosphorthioat,
O-Äthyl-O-(2,4-dichlorphenyl)-phenyl-phosphonothioat,
O,O-Dimethyl-O-(2,4,5-trichlorphenyl)-phosphorthioat,
O-Äthyl-O-(2,4,5-trichlorphenyl)-äthyl-phosphonothioat,
O,O-Dimethyl-O-(4-brom-2,5-dichlorphenyl)-phosphorthioat,
O,O-Dimethyl-O-(2,5-dichlor-4-jodphenyl)-phosphorthioat,
O,O-Dimethyl-O-(3-methyl-4-methylthiophenyl)-phosphorthioat,
O-Äthyl-O-(3-methyl-4-methylthiophenyl)-isopropyl-phosphoramidat,
O,O-Diäthyl-O-[p-(methylsulfinyl)-phenyl]-phosphorthioat,
O-Äthyl-S-phenyl-äthyl-phosphonodithioat,
O,O-Diäthyl-[2-chlor-1-(2,4-dichlorphenyl)-vinyl]-phosphat,
O,O-Dimethyl-[-2-chlor-1-(2,4,5-trichlorphenyl)]-vinyl-phosphat,
O,O-Dimethyl-S-(1'-phenyl)-äthylacetat-phosphordithioat,
Bis-(dimethylamino)-fluorphosphinoxid,
Octamethyl-pyrophosphoramid,
O,O,O,O-Tetraäthyldithio-pyrophosphat,
S-Chlormethyl-O,O-diäthyl-phosphordithioat,
O-Äthyl-S,S-dipropyl-phosphordithioat,
O,O-Dimethyl-O-2,2-dichlorvinyl-phosphat,

O,O-Dimethyl-1,2-dibrom-2,2-dichloräthylphosphat,
O,O-Dimethyl-2,2,2-trichlor-1-hydroxy-äthylphosphonat,
O,O-Dimethyl-S-[1,2-biscarbäthoxy-äthyl-(1)]-phosphordithioat,
O,O-Dimethyl-O-(1-methyl-2-carbmethoxy-vinyl)-phosphat,
O,O-Dimethyl-S-(N-methyl-carbamoyl-methyl)-phosphordithioat,
O,O-Dimethyl-S-(N-methyl-carbamoyl-methyl)-phosphorthioat,
O,O-Dimethyl-S-(N-methoxyäthyl-carbamoyl-methyl)-phosphordithioat,
O,O-Dimethyl-S-(N-formyl-N-methyl-carbamoylmethyl-phosphordithioat,
O,O-Dimethyl-O-[1-methyl-2-(methyl-carbamoyl)-vinyl]-phosphat,
O,O-Dimethyl-O-[(1-methyl-2-dimethylcarbamoyl)-vinyl]-phosphat,
O,O-Dimethyl-O-[(1-methyl-2-chlor-2-diäthylcarbamoyl)-vinyl]-phosphat,
O,O-Diäthyl-S-(äthylthio-methyl)-phosphordithioat,
O,O-Diäthyl-S-[(p-chlorphenylthio)-methyl]-phosphordithioat,
O,O-Dimethyl-S-(2-äthylthioäthyl)-phosphorthioat,
O,O-Dimethyl-S-(2-äthylthioäthyl)-phosphordithioat,
O,O-Dimethyl-S-(2-äthylsulfinyl-äthyl)-phosphorthioat,
O,O-Diäthyl-S-(2-äthylthio-äthyl)-phosphordithioat,
O,O-Diäthyl-S-(2-äthylsulfinyl-äthyl)-phosphorthioat,
O,O-Diäthyl-thiophosphoryliminophenyl-acetonitril,
O,O-Diäthyl-S-(2-chlor-1-phthalimidoäthyl)-phosphordithioat,
O,O-Diäthyl-S-[6-chlor-benzoxazolon-(2)-yl-(3)]-methyldithiophosphat,
O,O-Dimethyl-S-[2-methoxy-1,3,4-thiadiazol-5-[4H]-onyl-(4)-methyl]-phosphorditihioat,
O,O-Diäthyl-O-[3,5,6-trichlor-pyridyl-(2)]-phosphorthioat,
O,O-Diäthyl-O-(2-pyrazinyl)-phosphorthioat,
O,O-Diäthyl-O-[2-isopropyl-4-methyl-pyrimidinyl-(6)]-phosphorthioat,
O,O-Diäthyl-O-[2-(diäthylamino)-6-methyl-4-pyrimidinyl]-thionophosphat,
O,O-Dimethyl-S-(4-oxo-1,2,3-benzotriazin-3-[4H]-yl-methyl)-phosphordithioat,
O,O-Dimethyl-S-[4,6-diamino-1,3,5-triazin-2-yl)-methyl]-phosphordithioat,
O,O-Diäthyl-(1-phenyl-1,2,4-triazol-3-yl)-thionophosphat,
O,S-Dimethyl-phosphor-amido-thioat,
O,S-Dimethyl-N-acetyl-phosphoramidothioat,
γ-Hexachlorcyclohexan,
1,1-Di-(p-methoxyphenyl)-2,2,2-trichlor-äthan,
6,7,8,9,10,10-Hexachloro-1,5,5a,6,9,9a-hexahydro-6,9-methano-2,4,3-benzodioxa-thiepin-3-oxid,
Pyrethrine,
DL-2-Allyl-3-methyl-cyclopenten-(2)-on-(1)-yl-(4)-DL-cis,trans-chrysanthemat,
5-Benzyl-furyl-(3)-methyl-DL-cis,trans-chrysanthemat,
3-Phenoxybenzyl(±)-cis,trans-2,2-dimethyl-3-(2,2-dichlorvinyl)-cyclopropancarboxylat,
α-Cyano-3-phenoxybenzyl(±)-cis,trans-2,2-dimethyl-3-(2,2-dichlorvinyl)-cyclopropancarboxylat,
(s)-α-Cyano-3-phenoxybenzyl-cis(1R,3R)-2,2-dimethyl-3-(2,2-dibromvinyl)-cyclopropancarboxylat,
3,4,5,6-Tetrahydrophthalimidoäthyl-DL-cis,trans-chrysanthemat,
2-Methyl-5-(2-propinyl)-3-furylmethyl-chrysanthemat,
α-Cyano-3-phenoxybenzyl-α-isopropyl-4-chlorphenylacetat.

Die folgenden Beispiele belegen die biologische Wirkung der neuen Verbindungen. Vergleichsmittel ist der aus der US-PS 3 810 902 bekannte Wirkstoff

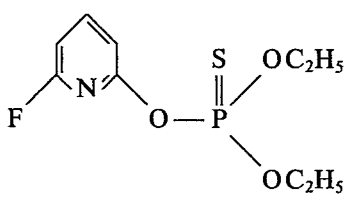

(A)

Die Numerierung der übrigen Wirkstoffe entspricht der der tabellarischen Auflistung.

## Beispiel A

### Kontaktwirkung auf Schaben (Blatta orientalis)

Der Boden eines 1-l-Einmachglases wird mit der acetonischen Lösung des Wirkstoffes behandelt. Nach dem Verdunsten des Lösungsmittels setzt man je Glas 5 adulte Schaben.

Die Mortalitätsrate wird nach 48 Stunden bestimmt.

| Wirkstoff Nr. | Wirkstoffmenge pro Glas [mg] | Mortalitätsrate [%] |
|---|---|---|
| 1 | 0,02 | 100 |
| 2 | 0,04 | 100 |
| 3 | 0,05 | 100 |
| 4 | 0,1 | 100 |
| A | 0,2 | 100 |
| A | 0,1 | <50 |

## Beispiel B

### Kontaktwirkung auf Stubenfliegen (Musca domestica); Applikationstest

4 Tage alten Imagines wird in leichter $CO_2$-Narkose 1 µl der acetonischen Lösung des Wirkstoffes auf das ventrale Abdomen appliziert. Hierzu wird eine Mikrometerspritze verwendet.

Je 20 Versuchstiere mit gleicher Behandlung werden dann in einen Folienbeutel (Volumen: ca. 500 ml) gebracht. Nach 4 Stunden werden die Tiere in Rückenlage ausgezählt, und die LD 50 wird graphisch ermittelt.

| Wirkstoff Nr. | LD 50 [µg/Fliege] |
|---|---|
| 1 | 0,055 |
| 2 | 0,12 |
| 3 | 0,04 |

## Beispiel C

### Fraß- und Kontaktwirkung auf Raupen der Kohlschabe (Plutella maculipennis)

Blätter von jungen Kohlpflanzen werden 3 Sekunden in die wäßrige Wirkstoffemulsion getaucht und nach kurzem Abtropfen auf einen angefeuchteten Filter in eine Petrischale gelegt. Das Blatt wird darauf mit 10 Raupen des 4. Stadiums belegt.

Nach 48 Stunden beurteilt man die Wirkung.

| Wirkstoff Nr. | Konzentration der Wirkstoff-emulsion [%] | Mortalitätsrate [%] |
|---|---|---|
| 1 | 0,002 | 100 |
| 2 | 0,002 | 80 |
| A | 0,004 | 80 |

## Beispiel D

### Kontaktwirkung auf Blattläuse (Aphis fabae); Spritzversuch

Getopfte Bohnenpflanzen (Vicia faba) mit starken Blattlauskolonien werden in der Spritzkammer mit den wäßrigen Wirkstoffaufbereitungen tropfnaß gespritzt.

Die Auswertung erfolgt nach 24 Stunden.

| Wirkstoff Nr. | Konzentration der Wirkstoff-aufbereitung [%] | Mortalitätsrate [%] |
|---|---|---|
| 1 | 0,01 | 100 |
| 2 | 0,01 | 100 |
| 3 | 0,001 | 80 |
| 4 | 0,025 | 100 |

## Beispiel E

### Kontaktwirkung auf Kornkäfer (Sitophilus granaria)

Petrischalen von 10 cm Durchmesser werden mit der acetonischen Wirkstofflösung ausgekleidet. Nach Verdunsten des Lösungsmittels belegt man die Schalen mit je 100 Kornkäfern. Nach 4 Stunden werden die Käfer in unbehandelte Gefäße überführt. Die Mortalitätsrate wird nach 24 Stunden ermittelt. Dabei wird festgestellt, wieviele Käfer in der Lage sind, nach diesem Zeitpunkt innerhalb 60 Minuten ein unbehandeltes Pappschälchen (Durchmesser: 40 mm, Höhe: 10 mm) zu verlassen.

| Wirkstoff Nr. | Wirkstoffmenge pro Petrischale [mg] | Mortalitätsrate [%] |
|---|---|---|
| 1 | 0,02 | 100 |
| 2 | 0,2 | 100 |
| 2 | 0,1 | 80 |
| 3 | 0,04 | 100 |
| 4 | 0,04 | 80 |
| A | 0,2 | 100 |
| A | 0,1 | <50 |

Beispiel F

Wirkung gegen Wurzelgallennematoden (Meloidogyne incognita)

Je 300 g einer stark mit Nematoden (Meloidogyne incognita) infizierten Komposterde werden mit 30 ml der wäßrigen Wirkstoffaufbereitung innig gemischt, in Plastiktöpfe von 8 cm Durchmesser gefüllt und mit einem jungen Tomatensetzling bepflanzt. Die Pflanzen stehen anschließend ca. 6 Wochen unter Gewächshausbedingungen bei einer Temperatur von 24°C. Anschließend wird die Vergallung der Wurzel beurteilt.

| Wirkstoff Nr. | Konzentration des Wirkstoffs im Boden [ppm] | |
|---|---|---|
| 2 | 25 | keine Gallenbildung |
| 4 | 25 | keine Gallenbildung |

**Patentansprüche für die Vertragsstaaten: BE, CH, LI, DE, FR, GB, IT, LU, NL, SE**

1. 6-Fluorpyridyl-(di)(thio)phosphorsäureester der Formel I

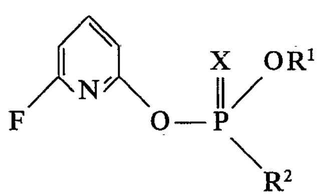

(I)

in der

X Sauerstoff oder Schwefel,
$R^1$ eine verzweigte oder unverzweigte Alkylgruppe mit bis zu 3 Kohlenstoffatomen und
$R^2$ eine verzweigte oder unverzweigte Alkylthiogruppe mit bis zu 6 Kohlenstoffatomen bedeuten.

2. O-Äthyl-S-n-propyl-O-(6-fluor-pyridyl-2)-thiophosphat.
3. O-Äthyl-S-i-butyl-O-(6-fluor-pyridyl-2)-phosphat.

4. Verfahren zur Herstellung von 6-Fluorpyridyl-(di)(thio)phosphorsäureestern der Formel I gemäß Anspruch 1, dadurch gekennzeichnet, daß man ein Fluorpyridylderivat der Formel II

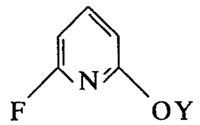

(II)

in der Y Wasserstoff, ein Alkaliion oder ein Äquivalent Erdalkaliion bedeutet, mit einem (Di)(Thio)Phosphorsäureesterchlorid der Formel III

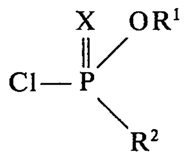

(III)

in der $R^1$, $R^2$ und X die im Anspruch 1 genannten Bedeutungen haben, gegebenenfalls in Anwesenheit eines Lösungs- oder Verdünnungsmittels bei Temperaturen von 0 bis 150°C umsetzt.

5. Schädlingsbekämpfungsmittel, enthaltend einen 6-Fluorpyridyl-(di)(thio)phosphorsäureester der Formel I gemäß Anspruch 1.

6. Insektizides und nematizides Mittel, enthaltend einen 6-Fluorpyridyl-(di)(thio)phosphorsäureester der Formel I gemäß Anspruch 1.

7. Verfahren zur Bekämpfung von Schädlingen, dadurch gekennzeichnet, daß man einen 6-Fluorpyridyl-(di)(thio)phosphorsäureester der Formel I gemäß Anspruch 1 auf die Schädlinge bzw. deren Lebensräume einwirken läßt.

**Patentansprüche für den Vertragsstaat: AT**

1. Schädlingsbekämpfungsmittel, enthaltend einen 6-Fluorpyridyl-(di)(thio)phosphorsäureester der Formel I

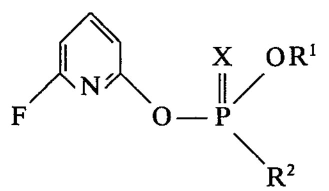

(I)

in der

X Sauerstoff oder Schwefel,
$R^1$ eine verzweigte oder unverzweigte Alkylgruppe mit bis zu 3 Kohlenstoffatomen und
$R^2$ eine verzweigte oder unverzweigte Alkylthiogruppe mit bis zu 6 Kohlenstoffatomen bedeuten.

2. Schädlingsbekämpfungsmittel, enthaltend O-Äthyl-S-n-propyl-O-(6-fluor-pyridyl-2)-thiophosphat.

3. Schädlingsbekämpfungsmittel, enthaltend O-Äthyl-S-i-butyl-O-(6-fluor-pyridyl-2)-phosphat.

4. Verfahren zur Herstellung von 6-Fluorpyridyl-(di)(thio)phosphorsäureestern der Formel I gemäß Anspruch 1, dadurch gekennzeichnet, daß man ein Fluorpyridylderivat der Formel II

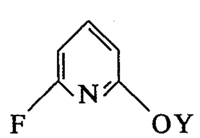

(II)

in der Y Wasserstoff, ein Alkaliion oder ein Äquivalent Erdalkaliion bedeutet, mit einem (Di)(Thio)Phosphorsäureesterchlorid der Formel III

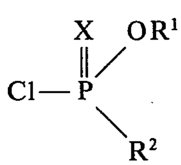

(III)

in der $R^1$, $R^2$ und X die im Anspruch 1 genannten Bedeutungen haben, gegebenenfalls in Anwesenheit eines Lösungs- oder Verdünnungsmittels bei Temperaturen von 0 bis 150° C umsetzt.

5. Verfahren zur Bekämpfung von Schädlingen, dadurch gekennzeichnet, daß man einen 6-Fluorpyridyl-(di)(thio)phosphorsäureester der Formel I gemäß Anspruch 1 auf die Schädlinge bzw. deren Lebensräume einwirken läßt.

**Claims for the Contracting states: BE, CH, LI, DE, FR, GB, IT, LU, NL, SE**

1. A 6-fluoropyridyl-(di)(thio)phosphoric acid ester of the formula I

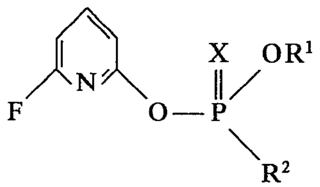

(I)

where X is oxygen or sulfur, $R^1$ is branched or linear alkyl of a maximum of 3 carbon atoms, and $R^2$ is branched or linear alkylthio of a maximum of 6 carbon atoms.

2. O-ethyl-S-n-propyl-O-(6-fluoropyrid-2-yl)-thiophosphate.

3. O-ethyl-S-isobutyl-O-(6-fluoropyrid-2- yl)-phosphate.

4. A process for the manufacture of 6-fluoropyridyl-(di)(thio)phosphoric acid esters of the formula I as claimed in claim 1, characterized in that a fluoropyridyl derivative of the formula II

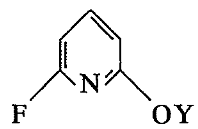

(II)

where Y denotes hydrogen, an alkali metal ion or one equivalent of an alkaline earth metal ion, is reacted with a (di)(thio)phosphoric acid ester chloride of the formula III

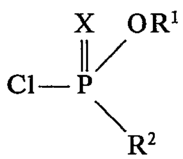

(III)

where $R^1$, $R^2$ and X have the meanings given in claim 1, in the presence or absence of a solvent or diluent and at from 0° to 150° C.

5. A pesticide containing a 6-fluoropyridyl-(di)(thio)phosphoric acid ester of the formula I as claimed in claim 1.

6. An insecticidal and nematocidal agent containing a 6-fluoropyridyl-(di)(thio)phosphoric acid ester of the formula I as claimed in claim 1.

7. A process for combating pests, characterized in that a 6-fluoropyridyl-(di)(thio)phosphoric acid ester of the formula I as claimed in claim 1 is allowed to act on the pests or their habitat.

**Claims for the Contracting state: AT**

1. A pesticide containing a 6-fluoropyridyl-(di)(thio)phosphoric acid ester of the formula I

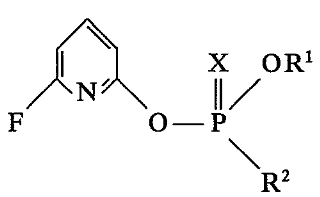

(I)

where X is oxygen or sulfur, $R^1$ is branched or linear alkyl of a maximum of 3 carbon atoms, and $R^2$ is branched or linear alkylthio of a maximum of 6 carbon atoms.

2. A pesticide containing O-ethyl-S-n-propyl-O-(6-fluoropyrid-2-yl)-thiophosphate.

3. A pesticide containing O-ethyl-S-isobutyl-O-(6-fluoropyrid-2-yl)-phosphate.

4. A process for the manufacture of 6-fluoropyridyl-(di)(thio)phosphoric acid esters of the formula I as claimed in claim 1, characterized in that a fluoropyridyl derivative of the formula II

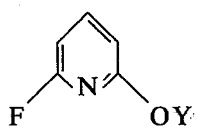

(II)

where Y denotes hydrogen, an alkali metal ion or one equivalent of an alkaline earth metal ion, is reacted with a (di)(thio)phosphoric acid ester chloride of the formula III

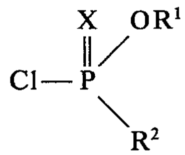

(III)

where $R^1$, $R^2$ and X have the meanings given in claim 1, in the presence or absence of a solvent or diluent and at from 0° to 150° C.

5. A process for combating pests, characterized in that a 6-fluoropyridyl-(di)(thio)phosphoric acid ester of the formula I as claimed in claim 1 is allowed to act on the pests or their habitat.

**Revendications pour les Etats contractants: BE, CH, LI, DE, FR, GB, IT, LU, NL, SE**

1. Ester 6-fluoropyridylique d'acide (di)(thio)phosphorique de formule I

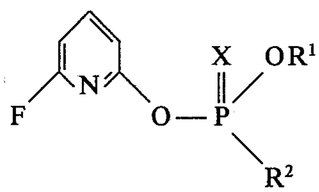

(I)

dans laquelle

X représente oxygène ou soufre,
$R^1$ un groupe alkyle ayant jusqu'à 3 atomes de carbone, ramifié ou non, et
$R^2$ un groupe alkylthio ayant jusqu'à 6 atomes de carbone, ramifié ou non.

2. O-éthyl-S-n-propyl-O-(6-fluoropyridyl-2)-thiophosphate.
3. O-éthyl-S-i-butyl-O-(6-fluoropyridyl-2)-phosphate.
4. Procédé de préparation d'ester 6-fluoropyridylique d'acide (di)(thio)phosphorique selon la revendication 1, caractérisé par le fait qu'on fait réagir, à des températures de 0 à 150°C, éventuellement en présence d'un solvant ou diluant, un dérivé fluoropyridylique de formule II

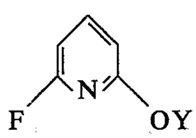

(II)

dans laquelle Y représente l'hydrogène, un ion alcalin ou un équivalent d'ion alcalinoterreux, avec un chlorure d'ester d'acide (di)(thio)phosphorique de formule III

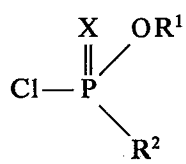

(III)

dans laquelle $R^1$, $R^2$ et X ont les significations données dans la revendication 1.

5. Pesticide contenant un ester 6-fluoropyridylique d'acide (di)(thio)phosphorique de formule I selon la revendication 1.

6. Agent insecticide et nématocide contenant un ester 6-fluoropyridylique d'acide (di)(thio)phosphorique selon la revendication 1.

7. Procédé de lutte contre les parasites, caractérisé par le fait qu'on fait agir sur les parasites ou leur biotope un ester 6-fluoropyridylique d'acide (di)(thio)phosphorique de formule I selon la revendication 1.

**Revendications pour l'Etat contractant: AT**

1. Pesticide contenant un ester 6-fluoropyridylique d'acide (di)(thio)phosphorique de formule I

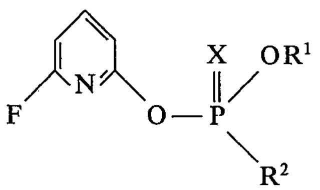

(I)

dans laquelle

X représente oxygène ou soufre,
$R^1$ un groupe alkyle ayant jusqu'à 3 atomes de carbone, ramifié ou non. et
$R^2$ un groupe alkylthio ayant jusqu'à 6 atomes de carbone, ramifié ou non.

2. Pesticide, contenant O-éthyl-S-n-propyl-O-(6-fluoropyridyl-2)-thiophosphate.

3. Pesticide, contenant O-éthyl-S-i-butyl-O-(6-fluoropyridyl-2)-phosphate.

4. Procédé de préparation d'ester 6-fluoropyridylique d'acide (di)(thio)phosphorique de formule I selon la revendication 1, caractérisé par le fait qu'on fait réagir, à des températures de 0 à 150°C, éventuellement en présence d'un solvant ou diluant, un dérivé fluoropyridylique de formule II

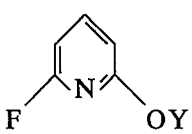

(II)

dans laquelle Y représente l'hydrogène, un ion alcalin ou un équivalent d'ion alcalinoterreux, avec un chlorure d'ester d'acide (di)(thio)phosphorique de formule III

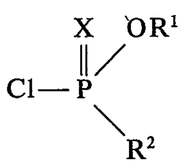

(III)

dans laquelle $R^1$, $R^2$ et X ont les significations données dans la revendication 1.

5. Procédé de lutte contre les parasites, caractérisé par le fait qu'on fait agir sur les parasites ou leur bistope, un ester 6-fluoropyridylique d'acide (di)(thio)phosphorique de formule I selon la revendication 1.